(19) Europäisches Patentamt
European Patent Office
Office européen des brevets

(11) Veröffentlichungsnummer: **0 122 553 B1**

(12) # EUROPÄISCHE PATENTSCHRIFT

(45) Veröffentlichungstag der Patentschrift: **26.02.86**

(51) Int. Cl.⁴: **C 07 C 145/02, A 01 N 41/06**

(21) Anmeldenummer: **84103780.7**

(22) Anmeldetag: **05.04.84**

(54) **N-Sulfenylierte Phenethylsulfonamide.**

(30) Priorität: **15.04.83 DE 3313718**

(43) Veröffentlichungstag der Anmeldung: **24.10.84 Patentblatt 84/43**

(45) Bekanntmachung des Hinweises auf die Patenterteilung: **26.02.86 Patentblatt 86/9**

(84) Benannte Vertragsstaaten: **AT BE CH DE FR GB IT LI NL SE**

(56) Entgegenhaltungen: **EP - A - 0 075 172**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., von-Bodelschwingh-Strasse 42, D-5060 Bergisch-Gladbach 2 (DE)**
Erfinder: **Reinecke, Paul, Dr., Lessingstrasse 11, D-5090 Leverkusen 3 (DE)**
Erfinder: **Brandes, Wilhelm, Dr., Eichendorfstrasse 3, D-5653 Leichlingen 1 (DE)**

**EP 0 122 553 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue N-sulfenylierte Phenethylsulfonamide, ein Verfahren zu ihrer Herstellung und ihre Verwendung als Schädlingsbekämpfungsmittel.

Es ist bekannt, daß N-Trihalogenmethyl-thio-Verbindungen als Fungizide in Landwirtschaft und Gartenbau verwendet werden können. So werden z.B. N-(Dichlorfluormethylthio)-N-ethyl-4-chlorbenzylsulfonamid, N-(Dichlorfluormethylthio)-N-methyl-2-trifluormethylbenzyl-sulfonamid, N-(Dichlorfluormethylthio)-N-phenyl-2-trifluormethylbenzylsulfonamid und N-(Dichlorfluormethylthio)-N-phenyl-2,6-dichlorbenzylsulfonamid zur Bekämpfung von Pilzkrankheiten eingesetzt (vgl. DE-OS 3 137 061).

Die Wirkung dieser Verbindungen ist jedoch unter gewissen Bedingungen, z.B. bei niedrigen Aufwandmengen, nicht immer ganz befriedigend.

Es wurden neue N-sulfenylierte Phenethylsulfonamide der allgemeinen Formel I

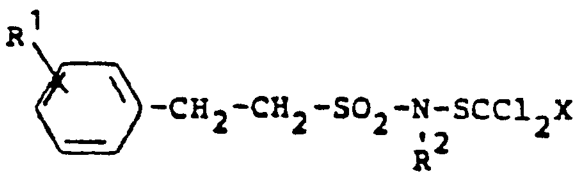

(I)

gefunden, in welcher

$R^1$ für Wasserstoff oder Chlor steht und

$R^2$ für Alkyl mit 1 bis 4 Kohlenstoffatomen, für Alkenyl mit 2 bis 5 Kohlenstoffatomen, für Alkoxyalkyl oder Alkylthioalkyl mit 1 bis 4 Kohlenstoffatomen je Alkylteil, für gegebenenfalls ein- bis dreifach durch Methyl oder Ethyl substituiertes Cycloalkyl mit 4 bis 6 Kohlenstoffatomen, für Aralkyl mit 1 bis 2 Kohlenstoffatomen im Alkylteil und 6 oder 10 Kohlenstoffatomen im Arylteil, für Phenyl oder für Naphthyl steht, wobei der Ring im Aralkyl, der Phenyl- oder Naphthylring gegebenenfalls einfach bis achtfach, gleich oder verschieden durch Fluor, Chlor, Brom, Nitro, Cyano, Methyl, Trifluormethyl, Methoxy, Ethoxy, Propoxy, Methylthio, Ethylthio, oder Propylthio substituiert ist und

X Fluor oder Chlor bedeutet.

Man erhält die N-sulfenylierten Phenethylsulfonamide der allgemeinen Formel I, wenn man Phenethylsulfonamide der allgemeinen Formel II

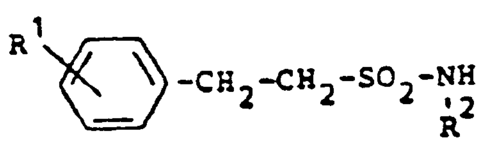

(II)

in welcher

$R^1$ und $R^2$ die oben angegebene Bedeutung haben,

mit einem Sulfenylchlorid der Formel III

$Cl\text{-}S\text{-}CCl_2X$ (III)

in welcher

X die oben angegebene Bedeutung hat,

in Gegenwart eines säurebindenden Mittels und gegebenenfalls in Gegenwart eines Verdünnungsmittels, umsetzt.

Die neuen N-sulfenylierten Phenethylsulfonamide der Formel (I) weisen starke fungizide Eigenschaften auf. Überraschenderweise besitzen die erfindungsgemäßen N-sulfenylierten Phenethylsulfonamide in verschiedenen Kulturen, z.B. in Getreide, eine höhere fungizide Wirksamkeit als die bekannten N-Trihalogenmethylthio-Verbindungen.

Im einzelnen seien neben den Herstellungsbeispielen folgende N-sulfenylierte Phenethylsulfonamide der

Formel 1 genannt: Die N-(Dichlorfluormethylsulfenyl)- und N-Trichlormethyl-sulfenyl-derivate von Phenylethylsulfonsäuren-n-propylamid, -isobutylamid, -cyclohexylamid, -benzylamid, -4-chloranilid, -3-trifluormethylanilid, die N-(Dichlorfluormethylsulfenyl)-derivate von 4-Chlorphen-ethylsulfonsäuremethylamid und 3-Nitrophenethylsulfonsäuremethylamid.

Verwendet man beispielsweise N-Methyl-2-phenethylsulfonamid und Fluordichlormethansulfenylchlorid als Ausgangskomponenten, so kann der Reaktionsverlauf durch das nachfolgende Formelschema wiedergegeben werden:

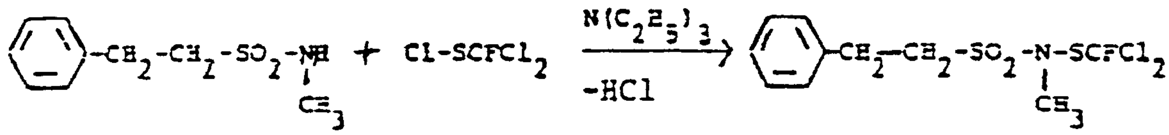


Die als Ausgangsstoffe benötigten Phenethylsulfonsäureamide sind durch die Formel II allgemein definiert. In dieser Formel haben $R^1$ und $R^2$ die Bedeutungen, die bei der Beschreibung der erfindungsgemäßen Verbindungen der Formel 1 für diese Substituenten bereits genannt worden sind.

Die Phenethylsulfonamide der Formel (II) sind bekannt oder in an sich bekannter Weise erhältlich, wenn man Phenethylsulfochloride der Formel IV

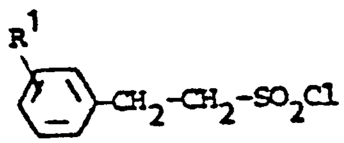

(IV)

in welcher

$R^1$ die oben angegebene Bedeutung hat,

mit einem primären Amin der Formel V

$R^2$-$NH_2$ (V)

in welcher

$R^2$ die oben angegebene Bedeutung hat,

gegebenenfalls in Gegenwart einer Base und eines Verdünnungsmittels umsetzt.

Die als Ausgangsstoffe benötigten Phenethyl-sulfochloride sind durch Formel IV allgemein definiert. Geeignete Phenethylsulfochloride der Formel IV sind beispielsweise Phenethyl- und 4-Chlorphenethylsulfochlorid. Die Verbindungen sind bekannt oder in an sich bekannter Weise aus entsprechenden Phenethylchloriden zugänglich (vgl. Houben-Weyl, Methoden der organischen Chemie, 4. Auflage, Band 9, Seite 395 (1955)).

Die als Ausgangsstoffe benötigten Amine sind durch Formel V allgemein definiert. In dieser Formel steht $R^2$ für die Bedeutungen, die bereits in Zusammenhang mit Formel 1 genannt worden sind.

Geeignete Amine der Formel V sind beispielsweise Methyl-, Ethyl-, Isopropyl-, Allyl-, tert.-Butyl-, Methoxyethyl-, Methylmercaptoethyl-, Cyclopentyl-, 4- Methylcyclohexyl-, Benzyl-, 4-Nitrobenzylamin, Anilin, 4-Chloranilin, 3-Fluor-anilin, 2-Toluidin, 3-Chlor-4-trifluormethylanilin und 1-Naphthylamin. Sie sind bekannte Verbindungen.

Verwendet man beispielsweise Phenethylsulfochlorid und Methylamin als Ausgangsstoffe, so kann der Reaktionsverlauf durch das folgende Formelschema wiedergegeben werden:

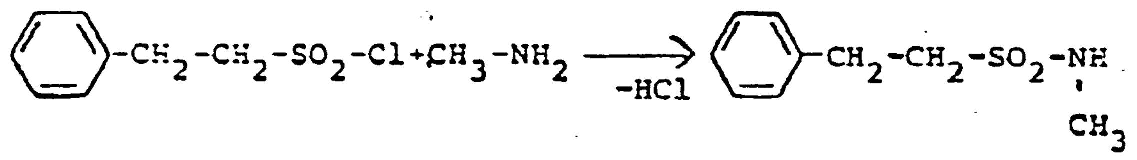

Bei der Durchführung des Verfahrens kommen als Verdünnungsmittel alle inerten organischen Lösungsmittel in Frage. Hierzu gehören vorzugsweise Kohlenwasserstoffe, wie Toluol, Chlorkohlenwasserstoffe, wie Methylenchlorid und Chlorbenzol, oder Ether, wie Dioxan.

Als säurebindende Mittel können anorganische Basen, wie Natriumhydroxid und Natriumcarbonat oder tert.-Amine, wie Pyridin und Triethylamin, verwendet werden.

Pro Mol Phenethylsulfochlorid der Formel IV wird etwa 1 Mol primäres Amin der Formel V eingesetzt. Die Reaktionstemperaturen können in einem größeren Bereich variiert werden. Im allgemeinen arbeitet man von 0 - 100° C, vorzugsweise von 20 - 50° C.

Das Verfahren kann wie folgt durchgeführt werden: Das Phenethylsulfochlorid der Formel IV wird in einem Verdünnungsmittel vorgelegt und auf 40°C erhitzt. Dazu wird das primäre Amin der Formel V portionsweise gegeben. Isolierung und Reinigung des Phenethylsulfonamids der Formel II erfolgt auf übliche Art und Weise.

Als Sulfenylchloride der Formel III können Fluordichlormethan-sulfenylchlorid und Trichlormethansulfenylchlorid eingesetzt werden.

Bei der Durchführung des erfindungsgemäßen Verfahrens kommen als Verdünnungsmittel alle inerten organischen Lösungsmittel in Frage. Hierzu gehören vorzugsweise Kohlenwasserstoffe, wie Toluol, Chlorkohlenwasserstoffe, wie Methylenchlorid und Chlorbenzol, oder Ether, wie Dioxan; auch Wasser kann verwendet werden.

Als säurebindende Mittel können anorganische Basen, wie Natriumhydroxid und Natriumcarbonat oder tert.-Amine, wie Pyridin und Triethylamin, verwendet werden.

Die Reaktionstemperaturen können in einem größeren Bereich variiert warden. Im allgemeinen arbeitet man von 0 bis 100° C, vorzugsweise von 20 bis 50° C.

Das erfindungsgemäße Verfahren wird wie folgt ausgeführt: Ein Phenethylsulfonsäureamid der Formel II und ein Sulfenylchlorid der Formel III werden in einem Verdünnungsmittel vorgelegt. Dazu wird bei Raumtemperatur der Säurebinder portionsweise gegeben, so daß die Reaktionstemperatur auf etwa 40°C ansteigt. Nach Beendigung der Umsetzung wird das N-sulfenylierte Phenethylsulfonamid der Formel (I) mit Wasser ausgefällt und auf übliche Art isoliert und gereinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind u.a. für den Gebrauch als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe zeigen u.a. eine gute fungizide Wirkung gegen Rost und Pyrenophora teres an Getreide und eine gute fungizide in-vitro-Wirkung.

Außerdem zeigen o.g. Wirkstoffe eine gute Wirkung gegen Oomyceten, wie z.B. gegen den Erreger der Kraut- und Fruchtfäule der Tomate (Phytopthora infestens) und gegen den Erreger des Apfelschorfs (Venturia inäqualis).

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon,

Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

**Herstellungsbeispiele**

**Beispiel 1**

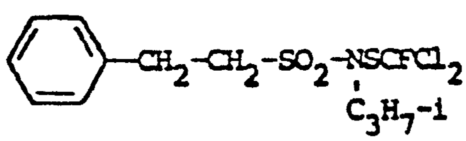

16 g (0,07 Mol) Phenethylsulfonsäure-N-isopropylamid werden in 100 ml Toluol gelöst und nach Zusatz von 8 g (0,08 Mol) Triethylamin mit 12 g (0,07 Mol) Dichlorfluormethansulfenylchlorid versetzt. Hierbei steigt die Temperatur bis etwa 40°C. Man wäscht die Reaktionslösung mit Wasser, trocknet die organische Phase und engt im Vakuum ein. Der Rückstand (21 g) wird aus Methanol umkristallisiert. Man erhält 18 g (71 % der Theorie) der gewünschten Verbindung mit einem Schmelzpunkt von 65°C.

In analoger Weise erhält man die Verbindungen der Formel (I)

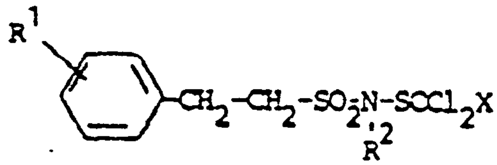

(I)

| Beispiel-Nr. | $R^1$ | $R^2$ | X | Schmelzpunkt [°C]; Brechungsindex [$n_D^{20}$] |
|---|---|---|---|---|
| 2 | H | $CH_3$ | F | 1,5513 |
| 3 | H | $C_2H_5$ | F | 1,5446 |
| 4 | H | $-CH_2-CH=CH_2$ | F | 1,5479 |

**Fortsetzung der Tabelle**

| Beispiel-Nr. | $R^1$ | $R^2$ | X | Schmelzpunkt [°C]; Brechungsindex [$n_D^{20}$] |
|---|---|---|---|---|
| 5 | H | $n-C_4H_9$ | F | 1,5347 |
| 6 | H | $tert.-C_4H_9$ | F | 58-59 |
| 7 | H | $C_6H_5$ | F | 132-133 |

**Herstellung der Ausgangsprodukte**

a)

a) 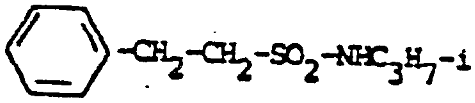

20,5 g (0,1 Mol) Phenethylsulfochlorid werden in 100 ml Aceton gelöst und bei Raumtemperatur tropfenweise mit 13 g (0,22 Mol) wasserfreiem Isopropylamin versetzt. Durch Eiswasserkühlung hält man die Temperatur auf 30°C. Nach Aufarbeitung über die Wasser-Toluolphasen erhält man 16 g (70 % der Theorie) des obigen Sulfonamids vom Schmelzpunkt 51-58 ° C.

Analog erhält man die Ausgangsverbindungen der Formel

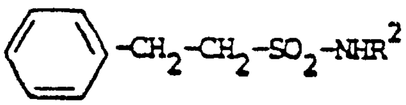

| Beispiel-Nr. | $R^2$ | Schmelzpunkt [°C] |
|---|---|---|
| b) | $CH_3$ | 60-61 |
| c) | $C_2H_5$ | 47-48 |
| d) | $-CH_2-CH=CH_2$ | 60-61 |
| e) | $n-C_4H_9$ | 67-68 |
| f) | tert.-$C_4H_9$ | 78-80 |
| g) | $C_6H_5$ | 74-76 |

Das Phenethylsulfochlorid ist in bekannter Weise aus Phenethylchlorid und Thioharnstoff zugänglich. Das bei dieser Reaktion erhältliche Phenethylisothiouroniumchlorid wird in wäßriger Lösung bei 5-20°C zum Phenethylsulfochlorid chloriert. $Kp_{0,1}$ 95-100°C; Fp. 30-33°C.

**Anwendungsbeispiele**

In den folgenden Beispielen werden die nachstehend aufgeführten Verbindungen als Vergleichssubstanzen eingesetzt:

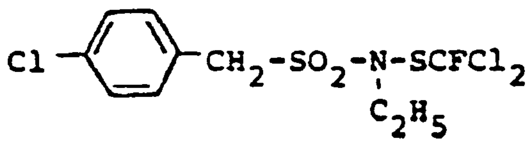

(A)

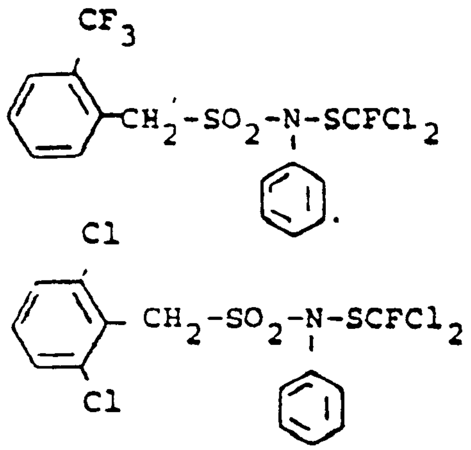

(B)

(C)

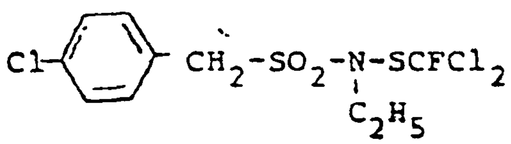

(D)

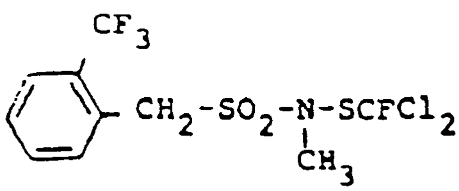

(E)

**Beispiel A**

Leptosphaeria nodorum-Test (Weizen)/ protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20° C und 100 % rel. Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 2, 3, 1 und 6.

**Beispiel B**

Phytophthora-Test (Tomate)/ protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20° C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen 2, 3, 1, 6.

**Beispiel C**

Venturia-Test (Apfel)/ protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 3, 1, 6.

**Patentansprüche**

1. N-sulfenylierte Phenethylsulfonamide der Formel (I)

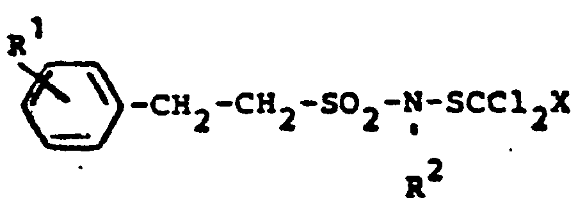

(I)

in welcher

$R^1$ für Wasserstoff oder Chlor steht,

$R^2$ für Alkyl mit 1 bis 4 Kohlenstoffatomen, für Alkenyl mit 2 bis 5 Kohlenstoffatomen, für Alkoxyalkyl oder Alkylthioalkyl mit 1 bis 4 Kohlenstoffatomen je Alkylteil, für gegebenenfalls ein- bis dreifach durch Methyl oder Ethyl substituiertes Cycloalkyl mit 4 bis 6 Kohlenstoffatomen, für Aralkyl mit 1 oder 2 Kohlenstoffatomen im Alkylteil und 6 oder 10 Kohlenstoffatomen im Arylteil, für Phenyl oder für Naphthyl steht, wobei der Ring im Aralkyl, der Phenyl- oder Naphthylring gegebenenfalls substituiert ist, einfach bis achtfach, gleich oder verschieden durch Fluor, Chlor, Brom, Nitro, Cyano, Methyl, Trifluormethyl, Methoxy, Ethoxy, Propoxy, Methylthio, Ethylthio oder Propylthio und

X für Fluor oder Chlor steht.

2. Verfahren zur Herstellung von N-sulfenylierten Phenethylsulfonamiden der Formel (I)

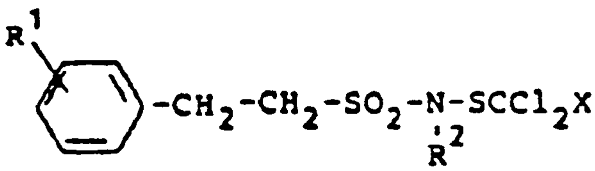

(I)

in welcher

$R^1$ für Wasserstoff oder Chlor steht,

$R^2$ für Alkyl mit 1 bis 4 Kohlenstoffatomen, für Alkenyl mit 2 bis 5 Kohlenstoffatomen, für Alkoxyalkyl oder Alkylthioalkyl mit 1 bis 4 Kohlenstoffatomen je Alkylteil, für gegebenenfalls ein- bis dreifach durch Methyl oder Ethyl substituiertes Cycloalkyl mit 4 bis 6 Kohlenstoffatomen, für Aralkyl mit 1 oder 2 Kohlenstoffatomen im Alkylteil und 6 oder 10 Kohlenstoffatomen im Arylteil, für Phenyl oder für Naphthyl steht, wobei der Ring im Aralkyl, der Phenyl- oder Naphthylring gegebenenfalls substituiert ist, einfach bis achtfach, gleich oder verschieden durch Fluor, Chlor, Brom, Nitro, Cyano, Methyl, Trifluormethyl, Methoxy, Ethoxy, Propoxy, Methylthio, Ethylthio oder Propylthio und

X für Fluor oder Chlor steht,

dadurch gekennzeichnet, daß man Phenethylsulfonamide der allgemeinen Formel (II)

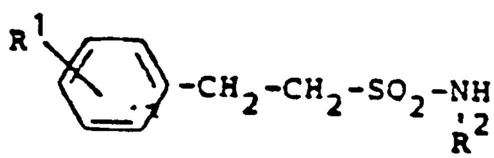

(II)

in welcher

$R^1$ und $R^2$ die oben angegebene Bedeutung haben,

mit einem Sulfenylchlorid der Formel

$Cl-S-CCl_2X$ (III)

in welcher

X die oben angegebene Bedeutung hat,

in Gegenwart eines säurebindenden Mittels und gegebenenfalls in Gegenwart eines Verdünnungsmittels, umsetzt.

3. Schädlingsbekämpfungsmittel, gekennzeichnet durch einen Gehalt an mindestens einem N-sulfenylierten Phenethylsulfonamid der Formel (I) gemäß Ansprüchen 1 oder 2.

4. Verwendung von N-sulfenylierten Phenethylsulfonamiden der Formel (I) gemäß Ansprüchen 1 oder 2 zur Bekämpfung von Schädlingen.

5. Verfahren zur Bekämpfung von Schädlingen, dadurch gekennzeichnet, daß man N-sulfenylierte Phenethylsulfonamide der Formel (I) gemäß Ansprüchen 1 oder 2 auf Schädlinge und/oder ihren Lebensraum einwirken läßt.

6. Verfahren zur Herstellung von Schädlingsbekämpfungsmitteln, dadurch gekennzeichnet, daß man N-sulfenylierte Phenethylsulfonamide der Formel (I) gemäß Anspruch 1 oder 2 mit Streckmitteln und/oder

oberflächenaktiven Mitteln vermischt.

## Claims

1. N-Sulphenylated phenethylsulphonamides of the formula (I)

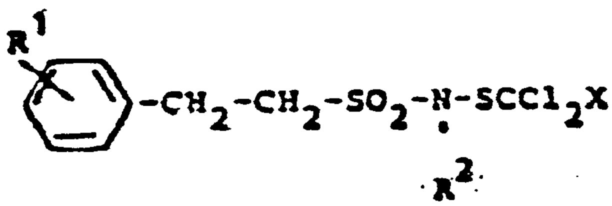

(I)

in which

R[1] represents hydrogen or chlorine,

R[2] represents alkyl with 1 to 4 carbon atoms, alkenyl with 2 to 5 carbon atoms, alkoxyalkyl or alkylthioalkyl with 1 to 4 carbon atoms per alkyl part, cycloalkyl which has 4 to 6 carbon atoms and is optionally mono- to trisubstituted by methyl or ethyl, aralkyl with 1 or 2 carbon atoms in the alkyl part and 6 or 10 carbon atoms in the aryl part, phenyl or naphthyl, the ring in the aralkyl, the phenyl ring or the naphthyl ring being optionally mono-to octasubstituted by identical or different substituents selected from fluorine, chlorine, bromine, nitro, cyano, methyl, trifluoromethyl, methoxy, ethoxy, propoxy, methylthio, ethylthio or propylthio, and X represents fluorine or chlorine.

2. Process for the preparation of N-sulphenylated phenethylsulphonamides of the formula (I)

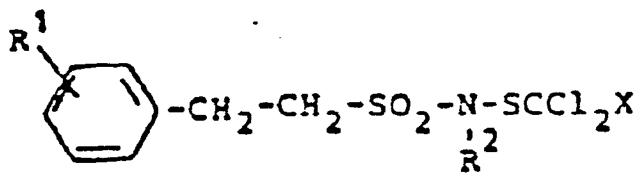

(I)

in which

R[1] represents hydrogen or chlorine,

R[2] represents alkyl with 1 to 4 carbon atoms, alkenyl with 2 to 5 carbon atoms, alkoxyalkyl or alkylthioalkyl with 1 to 4 carbon atoms per alkyl part, cycloalkyl which has 4 to 6 carbon atoms and is optionally mono- to trisubstituted by methyl or ethyl, aralkyl with 1 or 2 carbon atoms in the alkyl part and 6 or 10 carbon atoms in the aryl part, phenyl or naphthyl, the ring in the aralkyl, the phenyl ring or the naphthyl ring being optionally mono- to octasubstituted by identical or different substituents selected from fluorine, chlorine, bromine, nitro, cyano, methyl, trifluoromethyl, methoxy, ethoxy, propoxy, methylthio, ethylthio or propylthio, and

X represents fluorine or chlorine,

characterised in that phenethylsulphonamides of the general formula (II)

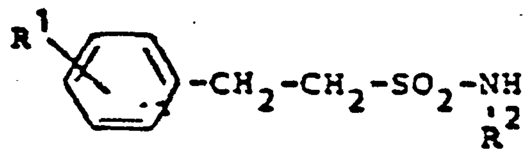

(II)

in which

$R^1$ and $R^2$ have the meaning given above, are reacted with a sulphenyl chloride of the formula (III)

$Cl-S-CCl_2X$ (III)

in which

X has the meaning given above, in the presence of an acid-binding agent and if appropriate in the presence of a diluent.

3. Agents for combating pests, characterised in that they contain at least one N-sulphenylated phenethylsulphonamide of the formula (I) according to Claims 1 or 2.

4. Use of N-sulphenylated phenethylsulphonamides of the formula (I) according to Claims 1 or 2 for combating pests.

5. Method of combating pests, characterised in that N-sulphenylated phenethylsulphonamides of the formula (I) according to Claims 1 or 2 are allowed to act on pests and/or their environment.

6. Process for the preparation of agents for combating pests, characterised in that N-sulphenylated phenethylsulphonamides of the formula (I) according to Claim 1 or 2 are mixed with extenders and/or surface-active agents.

**Revendications**

1. Phénéthylsulfonamides N-sulfénylés de formule (I)

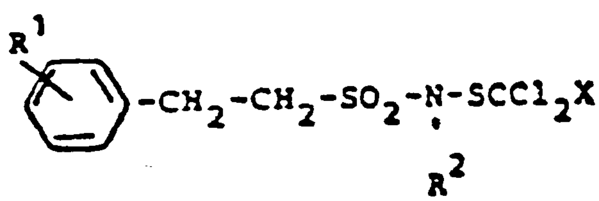

(I)

dans laquelle

$R^1$ désigne l'hydrogène ou le chlore,

$R^2$ est un groupe alkyle ayant 1 à 4 atomes de carbone, un groupe alcényle ayant 2 à 5 atomes de carbone, un groupe alkoxyalkyle ou alkylthioalkyle ayant 1 à 4 atomes de carbone par radical alkyle, un groupe cycloalkyle ayant 4 à 6 atomes de carbone, portant éventuellement 1 à 3 substituants méthyle ou éthyle, un groupe aralkyle ayant 1 ou 2 atomes de carbone dans la partie alkyle et 6 à 10 atomes de carbone dans la partie aryle, un groupe phényle ou un groupe naphtyle, le noyau du groupe aralkyle, le noyau phényle ou le noyau naphtyle étant éventuellement substitué une à huit fois par des substituants fluoro, chloro, bromo, nitro, cyano, méthyle, trifluorométhyle, méthoxy, éthoxy, propoxy, méthylthio, éthylthio ou propylthio égaux ou différents et

X représente le fluor ou le chlore.

2. Procédé de production de phénéthylsulfonamides N-sulfénylés de formule (I)

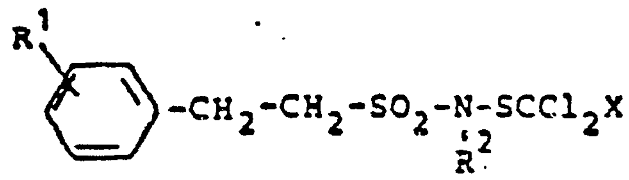

(I)

dans laquelle

$R^1$ désigne l'hydrogène ou le chlore,

$R^2$ est un groupe alkyle ayant 1 à 4 atomes de carbone, un groupe alcényle ayant 2 à 5 atomes de carbone, un groupe alkoxyalkyle ou alkylthioalkyle ayant 1 à 4 atomes de carbone par radical alkyle, un groupe cycloalkyle ayant 4 à 6 atomes de carbone portant éventuellement 1 à 3 substituants méthyle ou éthyle, un groupe aralkyle ayant 1 ou 2 atomes de carbone dans la partie alkyle et 6 ou 10 atomes de carbone dans la partie aryle, un groupe phényle ou un groupe naphtyle, le noyau du groupe aralkyle, le noyau phényle ou le noyau naphtyle étant éventuellement substitué une à huit fois par des substituants fluoro, chloro, bromo, nitro, cyano, méthyle, trifluorométhyle, méthoxy, éthoxy, propoxy, méthylthio, éthylthio ou propylthio égaux ou différents et

X représente du fluor ou du chlore,

caractérisé en ce qu'on fait réagir des phénéthylsulfonamides de formule générale (II)

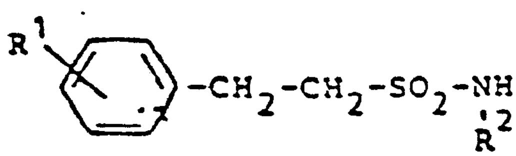

(II)

dans laquelle

$R^1$ et $R^2$ ont la définition indiquée ci-dessus, avec un chlorure de sulfényle de formule (III)

$Cl\text{-}S\text{-}CCl_2X$ (III)

dans laquelle

X à la définition indiquée ci-dessus,

en présence d'un accepteur d'acide et, le cas échéant, en présence d'un diluant.

3. Compositions pesticides, caractérisées par une teneur en au moins un phénéthylsulfonamide N-sulfénylé de formule (I) suivant les revendications 1 ou 2.

4. Utilisation de phénéthylsulfonamides N-sulfénylés de formule (I) suivant les revendications 1 ou 2 pour combattre des parasites.

5. Procédé pour combattre des parasites, caractérisé en ce qu'on fait agir sur les parasites et/ou sur leur milieu des phénéthylsulfonamides N-sulfénylés de formule (I) suivant les revendications 1 ou 2.

6. Procédé de préparation de compositions pour la lutte contre les parasites, caractérisé en ce qu'on mélange des phénéthylsulfonamides N-sulfénylés de formule (I) suivant la revendication 1 ou 2 avec des diluants et/ou des agents tensio-actifs.